# EUROPEAN PATENT APPLICATION

(11) **EP 2 108 635 A1**
(43) Date of publication of application: **14.10.2009**
(21) Application number: 08154405.8
(22) Date of filing: 11.04.2008
(51) Int. Cl.: C07C 1/24, C07C 11/04, C07C 11/06, C07C 11/08

(54) **Process to make olefins from ethanol**

(71) Applicant: Total Petrochemicals Research Feluy, 7181 Seneffe (Feluy) (BE)
(72) Inventor: Minoux, Delphine, 7181 Familleureux (BE); Nesterenko, Nikolai, 1400 Nivelles (BE); Vermeiren, Walter, 3530 Houthalen (BE); Van Donk, Sander, 1180 Uccle (BE); Grasso, Giacomo, 1060 Bruxelles (BE); Dath, Jean-Pierre, 7970 Beloeil (BE)
(74) Representative: Neel, Henry

(57) **Abstract**

The present invention relates to a process for the conversion of ethanol to make essentially ethylene and propylene, comprising:
a) introducing in a reactor (A) a stream comprising ethanol under a partial pressure at least about 0.2 MPa,
b) contacting said stream with a catalyst (A1) in said reactor (A) at conditions effective to convert at least a portion of the ethanol to ethylene, propylene and olefins having 4 carbon atoms or more (C4+ olefins),
c) recovering from said reactor an effluent comprising:
ethylene, C4+ fraction,
propylene and various hydrocarbons,
water,

d) fractionating said effluent of step c) to remove water and unconverted ethanol, to get a stream (D) comprising ethylene and C4+ olefins,
e) introducing at least a part of said stream (D) optionally mixed with a stream (D1) comprising C4+ olefins in an OCP reactor under the condition that said mixture (D)+(D1) comprises at least 10wt% of C4+ olefins,
f) contacting said stream comprising at least a part of (D) and the optional (D1) in said OCP reactor with a catalyst which is selective towards light olefins in the effluent, to produce an effluent with an olefin content of lower molecular weight than that of the feedstock,
g) fractioning said effluent of step f) to produce at least an ethylene stream, a propylene stream and a fraction consisting essentially of hydrocarbons having 4 carbon atoms or more.

## Description

### [Field of the invention]

The present invention relates to the conversion of ethanol to a mixture of ethylene, propylene and heavier olefins. The limited supply and increasing cost of crude oil has prompted the search for alternative processes for producing hydrocarbon products such as ethylene. Ethanol can be obtained by fermentation of carbohydrates. Made up of organic matter from living organisms, biomass is the world's leading renewable energy source. In an advantageous embodiment the heavier olefins and a part of the ethylene are recovered and cracked on a catalyst to give more propylene.

### [Background of the invention]

US 4207424 describes a process for the catalytic dehydration of alcohols to form unsaturated organic compounds in which an alcohol is dehydrated in the presence of alumina catalysts which are pre-treated with an organic silylating agent at elevated temperature. Example 12 relates to ethanol, the pressure is atmospheric, the WHSV is 1.2 h⁻¹ and shows only a conversion increase by comparison with the same alumina but having not been pretreated.

US 4302357 relates to an activated alumina catalyst employed in a process for the production of ethylene from ethanol through a dehydration reaction. In the description LHSV of ethanol is from 0.25 to 5 h⁻¹ and preferably from 0.5 to 3 h⁻¹. The examples are carried out at 370°C, a pressure of 10 Kg/cm² and LHSV of 1 h⁻¹, ethylene yield is from 65 to 94%.

Process Economics Reviews PEP' 79-3 (SRI international) of December 1979 describes the dehydration of an ethanol-water (95/5 weight %) mixture on a silica-alumina catalyst in a tubular fixed bed at 315-360°C, 1.7 bar absolute and a WHSV (on ethanol) of 0.3 h⁻¹. The ethanol conversion is 99% and the ethylene selectivity is 94.95%. It also describes the dehydration of an ethanol-water (95/5 weight %) mixture on a silica-alumina catalyst in a fluidized bed at 399°C, 1.7 bar absolute and a WHSV (on ethanol) of 0.7 h⁻¹. The ethanol conversion is 99.6% and the ethylene selectivity is 99.3%.

US 4232179 relates to the preparation of ethylene, based on a process for dehydrating ethyl alcohol. More particularly, the object of said prior art is the production of ethylene in the presence of catalysts, using adiabatic reactors and a high temperature. Such adiabatic reactors may be used in parallel or may be arranged in series or arranged in assemblies of parallel series, or still only a single reactor may be used. The ratio between the sensible heat carrying stream and the feed may range from 0.2:1 to 20:1, but preferably shall be comprised within the range from 0.2:1 to 10:1. On the other hand the space velocity may range between 10 and 0.01 g/h of ethyl alcohol per gram of catalyst, depending on the desired operation severity, the range between 1.0 and 0.01 g/h/g being particularly preferred. In the examples the catalysts are silica alumina, the WHSV on ethanol is from 0.07 to 0.7, the ratio of steam to ethanol is from 3 to 5. The pressure ranges from 0.84 to 7 kg/cm² gauge.

EP 22640 relates to improved zeolite catalysts, to methods of producing such catalysts, and to their use in the conversion of ethanol and ethylene to liquid and aromatic hydrocarbons, including the conversion of ethanol to ethylene. More particularly this prior art relates to the use of zeolite catalysts of Si/Al ratio from 11 to 24 (in the examples) such as the ZSM and related types in the conversion reaction of aqueous and anhydrous ethanol to ethylene, of aqueous ethanol to higher hydrocarbons, and of ethylene into liquid and aromatic hydrocarbons. WHSV ranges from 5.3 to 6 h⁻¹, in dehydration to ethylene the reactor temperature is from 240 to 290°C. The pressure ranges from 1 to 2 atmospheres.

US 4727214 relates to a process for converting anhydrous or aqueous ethanol into ethylene wherein at least one catalyst of the crystalline zeolite type is used, said catalyst having, on the one hand, channels or pores formed by cycles or rings of oxygen atoms having 8 and/or 10 elements or members and an atomic Si/Al ratio of less than about 20.. In the examples the temperature is from 217 to 400°C, the pressure atmospheric and the WHSV 2.5 h⁻¹.

US 4847223 describes a catalyst comprising from 0.5 to 7% by weight of trifluoromethanesulfonic acid incorporated onto an acid-form pentasil zeolite having a Si/Al atomic ratio ranging from 5 to 54 and a process for producing same. Also within the scope of said prior art is a process for the conversion of dilute aqueous ethanol to ethylene comprising: flowing said ethanol through a catalyst comprising from 0.5 to 7% by weight of trifluoromethanesulfonic acid incorporated onto an acid-form pentasil zeolite having a Si/Al atomic ratio range from 5 to 54 at a temperature ranging from 170° to 225° C, atmospheric pressure and recovering the desired product. The WHSV is from 1 to 4.5 h⁻¹. The zeolites which are directly concerned by said prior art belong to the family called ZSM or pentasil zeolite family namely ZSM-5 and ZSM-11 type zeolites.

US 4873392 describes a process for converting diluted ethanol to ethylene which comprises heating an ethanol-containing fermentation broth thereby to vaporize a mixture of ethanol and water and contacting said vaporized mixture with a ZSM-5 zeolite catalyst selected from the group consisting of :
- a ZSM-5 zeolite having a Si/Al atomic ratio of from 5 to 75 which has been treated with steam at a temperature ranging from 400 to 800°C for a period of from 1 to 48 hours;
- a ZSM-5 zeolite having a Si/Al atomic ratio of from 5 to 50 and wherein La or Ce ions have been incorporated in a weight percentage of 0.1 to 1.0% by ion exchange or in a weight percentage ranging from 0.1 to 5% by impregnation, and
- a ZSM-5 zeolite having a Si/Al of from 5 to 50 and impregnated with a 0.5 to 7 wt % of trifluoromethanesulfonic acid, and recovering the ethylene thus produced.

In ex 1 the catalyst is a steamed ZSM-5 having a Si/Al ratio of 21, the aqueous feed contains 10 w% of ethanol and 2 w% of glucose, the temperature is 275°C, the WHSV is from 3.2 to 38.5 h⁻¹. The ethylene yield decreases with the increase of WHSV. The ethylene yield is 99.4% when WHSV is 3.2 h⁻¹ and 20.1% when WHSV is 38.5 h⁻¹.
In ex 2 a ZSM-5 having a Si/Al ratio of 10 is compared with the same but on which La or Ce ions have been incorporated. The aqueous feed contains 10 w% of ethanol and 2 w% of glucose, the temperature is from 200°C to 225°C, the WHSV is 1 h⁻¹ and the best ethylene yield is 94.9%.
In ex 3 the catalyst is a ZSM-5 having a Si/Al ratio of 10 on which trifluoromethanesulfonic acid has been incorporated, the aqueous feed contains 10 w% of ethanol and 2 w% of glucose, the temperature is from 180°C to 205°C, the WHSV is 1 h⁻¹. The ethylene yield increases with temperature (73.3% at 180°C, 97.2% at 200°C) and then decreases (95.8% at 205°C). Pressure is not mentionned in the examples.

US 4670620 describes ethanol dehydration to ethylene on ZSM-5 catalysts. In a preferred embodiment the catalysts used according to this prior art are of the ZSM-5 type and preferably at least partially under hydrogen form. In the examples the catalyst is a ZSM-5 or a ZSM-11 having a SI/AI ratio of 40 to 5000 (ex 13), the LHSV is from 0.1 to 1.8 h⁻¹, the pressure atmospheric and the temperature from 230°C to 415°C.

WO 2007083241 A2 describes a production method for propylene consisting in ethanol convertion into propylene by continuously reacting ethanol on a catalyst. The solid acid catalyst is **characterized in that** the kinetic constant k of the butane cracking reaction on the catalyst at 500°C is 0.1 to 30 (cm³/min⁺g), and the solid acid catalyst is used in the production method for propylene. The solid acid catalyst is **characterized in that** the aperture diameter of pores formed in surfaces of the catalyst is 0.3 to 1.0 nm, and the solid acid catalyst is used in the production method for propylene. Furthermore, a regeneration method for a catalyst is **characterized in that** a heating treatment in an oxygen atmosphere is performed on a catalyst that has been used to produce propylene in the production method for propylene of the invention.

WO2007055361 A1 describes a method of production propylene containing bio-mass-origin carbon. Ethanol obtained from a commonly employed biomass source contains impurities other than water. In the case of obtaining ethylene therefrom by a dehydration reaction, these impurities per se or decomposition products thereof contaminate the ethylene and exert undesirable effects on the activity of a metathesis catalyst. Said prior art describes a method of producing propylene characterized by comprising converting ethanol, which is obtained from such a biomass, into ethylene via a dehydration reaction, separating the ethylene from the water thus formed, purifying the ethylene thus separated by adsorbing by an adsorption column packed with an adsorbent, and conducting a metathesis reaction together with a material containing n-butene. Thus, propylene with reduced environmental burdens, which contains carbon originating in the biomass, can be efficiently produced without lowering the catalytic activity.

It has now been discovered that (bio)ethanol can be converted to (bio)propylene by process comprising the conversion of ethanol in a first low temperature reaction zone (advantageously around 300°C-450°C) to hydrocarbons mixture containing substantially a mixture of ethylene, propylene and heavier olefins, on a dehydration/oligmerization type catalyst under the first reaction conditions. Then, advantageously propylene is recovered, water is optionally extracted with the rest of non-converted oxygenates, the heavier olefins and the ethylene are subjected to a contact in a second high temperature (advantageously around 450°C-600°C) reaction zone with olefin cracking catalyst, also referred as an OCP catalyst (Olefin Cracking Process) to give a stream with a high propylene content.

### [Brief summary of the invention]

The present invention relates to a process for the conversion of ethanol to make essentially ethylene and propylene, comprising :
a) introducing in a reactor (A) (also called the first low temperature reaction zone) a stream comprising ethanol under a partial pressure at least about 0.2 MPa, optionally water, optionally an inert component,
b) contacting said stream with a catalyst (A1) in said reactor (A) at conditions effective to convert at least a portion of the ethanol to essentially ethylene, propylene and olefins having 4 carbon atoms or more (C4+ olefins),
c) recovering from said reactor an effluent comprising : ethylene and C4+ fraction containng mainly olefins having 4 carbon atoms or more (C4+ olefins), propylene and various hydrocarbons, water, optionally unconverted ethanol and the optional inert component of step a),
d) fractionating said effluent of step c) to remove water, unconverted ethanol, optionally the inert component, optionally the propylene and optionally the whole or a part of the various hydrocarbons to get a stream (D) comprising essentially ethylene, olefins having 4 carbon atoms or more (C4+ olefins) and optionally the inert component,
e) introducing at least a part of said stream (D) optionally mixed with a stream (D1) comprising olefins having 4 carbon atoms or more (C4+ olefins) in a OCP reactor (also called the second high temperature reaction zone) under the condition that said mixture (D)+(D1) comprises at least 10wt% of C4+ olefins,
f) contacting said stream comprising at least a part of (D) and the optional (D1) in said OCP reactor with a catalyst which is selective towards light olefins in the effluent, to produce an effluent with an olefin content of lower molecular weight than that of the feedstock,
g) fractionating said effluent of step f) to produce at least an ethylene stream, a propylene stream and a fraction consisting essentially of hydrocarbons having 4 carbon atoms or more, optionally recycling ethylene in whole or in part at the inlet of the OCP reactor of step f), or at the inlet of the reactor (A) or in part at the inlet of the OCP reactor of step f) and in part at the inlet of the reactor (A), optionally recycling the fraction consisting essentially of hydrocarbons having 4 carbon atoms or more at the inlet of the OCP reactor.

According to a second embodiment the present invention relates to a process for the conversion of ethanol to make essentially ethylene, olefins having 4 carbon atoms or more (C4+ olefins) and minor amounts of propylene, comprising :
a) introducing in a reactor (A) a stream comprising ethanol under a partial, pressure of at least 0.2 MPa, optionally water, optionally an inert component,
b) contacting said stream with a catalyst in said reactor (A) at conditions effective to convert at least a portion of the ethanol to essentially ethylene and C4+ fraction containng mainly olefins having 4 carbon atoms or more (C4+ olefins),
c) recovering from said reactor an effluent comprising : ethylene and olefins having 4 carbon atoms or more (C4+ olefins), propylene and various hydrocarbons, water, optionally unconverted ethanol and the optional inert component of step a),
   Wherein the catalyst is :
   - a crystalline silicate having a ratio Si/Al of at least about 100, or
   - a dealuminated crystalline silicate, or
   - a phosphorus modified zeolite, the temperature ranges from 280°C to 500°C.

### [Detailed description of the invention]

**As regards the stream introduced at step a)** the inert component is any component provided there is no adverse effect on the catalyst. Because the dehydration is endothermic the inert component can be used to bring energy. By way of examples the inert component is selected among the saturated hydrocarbons having up to 10 carbon atoms, naphtenes, nitrogen and CO2. Advantageously it is a saturated hydrocarbon or a mixture of saturated hydrocarbons having from 3 to 7 carbon atoms, more advantageously having from 4 to 6 carbon atoms and is preferably pentane. An example of inert component can be any individual saturated compound, a synthetic mixture of the individual saturated compounds as well as some equilibrated refinery streams like straight naphtha, butanes etc. Advantageously the inert component is a saturated hydrocarbon having from 3 to 6 carbon atoms and is preferably pentane. The weight proportions of respectively alcohol, water and inert component are, for example, 5-100/0-95/0-95 (the total being 100). The stream (A) can be liquid or gaseous.

**As regards the reactor (A),** it can be a fixed bed reactor, a moving bed reactor or a fluidized bed reactor. A typical fluid bed reactor is one of the FCC type used for fluidized-bed catalytic cracking in the oil refinery. A typical moving bed reactor is of the continuous catalytic reforming type. The dehydration may be performed continuously in a fixed bed reactor configuration using a pair of parallel "swing" reactors. The various preferred catalysts of the present invention have been found to exhibit high stability. This enables the dehydration process to be performed continuously in two parallel "swing" reactors wherein when one reactor is operating, the other reactor is undergoing catalyst regeneration. The catalyst of the present invention also can be regenerated several times.

**As regards the pressure in steps a) and b),** it can be any pressure provided the partial pressure of ethanol is above about 0.2 MPa absolute, advantageously from 0.2 MPa to 3 MPa absolute, more advantageously from 0.35 MPa to 1 MPa absolute and preferably from 0.4 MPa to 1 MPa absolute. "above about 0.2 MPa" means that 0.2 is not a strict limit but a pressure enough to produce a significant amount of olefins having 4 carbon atoms or more (C4+ olefins).

**As regards the temperature in step b),** it ranges from 280°C to 500°C, advantageously from 280°C to 450°C, more advantageously from 300°C to 450°C and preferably from 330°C to 400°C.

**As regards the WHSV of ethanol in step b),** it ranges from 0.1 to 20 h⁻¹, advantageously from 0.4 to 20 h⁻¹, more advantageously from 0.5 to 15 h⁻¹, preferably from 0.7 to 12 h⁻¹. In a specific embodiment the WHSV of the ethanol in step b) ranges advantageously from 2 to 20 h⁻¹, more advantageously from 4 to 20 h⁻¹, preferably from 5 to 15 h⁻¹, more preferably from 7 to 12 h⁻¹.

**As regards the catalyst (A1) of step b),** it can be any acid catalyst capable to cause the conversion of ethanol to hydrocarbon under above said conditions. By way of example, zeolites, modified zeolites, silica-alumina, alumina, silico-alumophosphates can be cited. Examples of such catalysts are cited in the above prior art.

**According to a first advantageous embodiment the catalyst (A1) is** a crystalline silicate containing advantageously at least one 10 members ring into the structure. It is by way of example of the MFI (ZSM-5, silicalite-1, boralite C, TS-1), MEL (ZSM-11, silicalite-2, boralite D, TS-2, SSZ-46), FER (Ferrierite, FU-9, ZSM-35), MTT (ZSM-23), MWW (MCM-22, PSH-3, ITQ-1, MCM-49), TON (ZSM-22, Theta-1, NU-10), EUO (ZSM-50, EU-1), MFS (ZSM-57) and ZSM-48 family of microporous materials consisting of silicon, aluminium, oxygen and optionally boron. Advantageously in said first embodiment the catalyst (A1) is a crystalline silicate having a ratio Si/Al of at least about 100 or a dealuminated crystalline silicate.

The crystalline silicate having a ratio Si/Al of at least about 100 is advantageously selected among the MFI and the MEL.

The crystalline silicate having a ratio Si/Al of at least about 100 and the dealuminated crystalline silicate are essentially in H-form. It means that a minor part (less than about 50 %) can carry metallic compensating ions e.g. Na, Mg, Ca, La, Ni, Ce, Zn, Co.

The dealuminated crystalline silicate is advantageously such as about 10% by weight of the aluminium is removed. Such dealumination can be done by any conventional techniques known per se but is advantageously made by a steaming optionally followed by a leaching. The crystalline silicate having a ratio Si/Al of at least about 100 can be synthetized as such or it can be prepared by dealumination of a crystalline silicate at conditions effective to obtain a ratio Si/Al of at least about 100. Such dealumination is advantageously made by a steaming optionally followed by a leaching.

The three-letter designations "MFI" and "MEL" each representing a particular crystalline silicate structure type as established by the Structure Commission of the International Zeolite Association.

Examples of a crystalline silicate of the MFI type are the synthetic zeolite ZSM-5 and silicalite and other MFI type crystalline silicates known in the art. Examples of a crystalline silicate of the MEL family are the zeolite ZSM-11 and other MEL type crystalline silicates known in the art. Other examples are Boralite D and silicalite-2 as described by the International Zeolite Association (Atlas of zeolite structure types, 1987, Butterworths). The preferred crystalline silicates have pores or channels defined by ten oxygen rings and a high silicon/aluminium atomic ratio.

Crystalline silicates are microporous crystalline inorganic polymers based on a framework of XO₄ tetrahedra linked to each other by sharing of oxygen ions, where X may be trivalent (*e*.*g*. Al,B,...) or tetravalent (*e*.*g*. Ge, Si,...). The crystal structure of a crystalline silicate is defined by the specific order in which a network of tetrahedral units are linked together. The size of the crystalline silicate pore openings is determined by the number of tetrahedral units, or, alternatively, oxygen atoms, required to form the pores and the nature of the cations that are present in the pores. They possess a unique combination of the following properties: high internal surface area; uniform pores with one or more discrete sizes; ion exchangeability; good thermal stability; and ability to adsorb organic compounds. Since the pores of these crystalline silicates are similar in size to many organic molecules of practical interest, they control the ingress and egress of reactants and products, resulting in particular selectivity in catalytic reactions. Crystalline silicates with the MFI structure possess a bidirectional intersecting pore system with the following pore diameters: a straight channel along [010]:0.53-0.56 nm and a sinusoidal channel along [100]:0.51-0.55 nm. Crystalline silicates with the MEL structure possess a bidirectional intersecting straight pore system with straight channels along [100] having pore diameters of 0.53-0.54 nm.

In this specification, the term "silicon/aluminium atomic ratio" or "silicon/aluminium ratio" is intended to mean the Si/Al atomic ratio of the overall material, which may be determined by chemical analysis. In particular, for crystalline silicate materials, the stated Si/Al ratios apply not just to the Si/Al framework of the crystalline silicate but rather to the whole material.

In a specific embodiment the catalyst preferably has a high silicon/aluminium atomic ratio, of at least about 100, preferably greater than about 150, more preferably greater than about 200, whereby the catalyst has relatively low acidity. The acidity of the catalyst can be determined by the amount of residual ammonia on the catalyst following contact of the catalyst with ammonia which adsorbs to the acid sites on the catalyst with subsequent ammonium desorption at elevated temperature measured by differential thermogravimetric analysis. Preferably, the silicon/aluminium ratio (Si/Al) ranges from about 100 to about 1000, most preferably from about 200 to about 1000. Such catalysts are known per se.

In a specific embodiment the crystalline silicate is steamed to remove aluminium from the crystalline silicate framework. The steam treatment is conducted at elevated temperature, preferably in the range of from 425 to 870°C, more preferably in the range of from 540 to 815°C and at atmospheric pressure and at a water partial pressure of from 13 to 200kPa. Preferably, the steam treatment is conducted in an atmosphere comprising from 5 to 100% steam. The steam atmosphere preferably contains from 5 to 100 vol% steam with from 0 to 95vol% of an inert gas, preferably nitrogen. A more preferred atmosphere comprises 72 vol% steam and 28 vol% nitrogen *i*.*e*. 72kPa steam at a pressure of one atmosphere. The steam treatment is preferably carried out for a period of from 1 to 200 hours, more preferably from 20 hours to 100 hours. As stated above, the steam treatment tends to reduce the amount of tetrahedral aluminium in the crystalline silicate framework, by forming alumina.

In a more specific embodiment the crystalline silicate catalyst is dealuminated by heating the catalyst in steam to remove aluminium from the crystalline silicate framework and extracting aluminium from the catalyst by contacting the catalyst with a complexing agent for aluminium to remove from pores of the framework alumina deposited therein during the steaming step thereby to increase the silicon/aluminium atomic ratio of the catalyst. The catalyst having a high silicon/aluminium atomic ratio for use in the catalytic process of the present invention is manufactured by removing aluminium from a commercially available crystalline silicate. By way of example a typical commercially available silicalite has a silicon/aluminium atomic ratio of around 120. In accordance with the present invention, the commercially available crystalline silicate is modified by a steaming process which reduces the tetrahedral aluminium in the crystalline silicate framework and converts the aluminium atoms into octahedral aluminium in the form of amorphous alumina. Although in the steaming step aluminium atoms are chemically removed from the crystalline silicate framework structure to form alumina particles, those particles cause partial obstruction of the pores or channels in the framework. This could inhibit the dehydration process of the present invention. Accordingly, following the steaming step, the crystalline silicate is subjected to an extraction step wherein amorphous alumina is removed from the pores and the micropore volume is, at least partially, recovered. The physical removal, by a leaching step, of the amorphous alumina from the pores by the formation of a water-soluble aluminium complex yields the overall effect of de-alumination of the crystalline silicate. In this way by removing aluminium from the crystalline silicate framework and then removing alumina formed there from the pores, the process aims at achieving a substantially homogeneous de-alumination throughout the whole pore surfaces of the catalyst. This reduces the acidity of the catalyst. The reduction of acidity ideally occurs substantially homogeneously throughout the pores defined in the crystalline silicate framework. Following the steam treatment, the extraction process is performed in order to de-aluminate the catalyst by leaching. The aluminium is preferably extracted from the crystalline silicate by a complexing agent which tends to form a soluble complex with alumina. The complexing agent is preferably in an aqueous solution thereof. The complexing agent may comprise an organic acid such as citric acid, formic acid, oxalic acid, tartaric acid, malonic acid, succinic acid, glutaric acid, adipic acid, maleic acid, phthalic acid, isophthalic acid, fumaric acid, nitrilotriacetic acid, hydroxyethylenediaminetriacetic acid, ethylenediaminetetracetic acid, trichloroacetic acid trifluoroacetic acid or a salt of such an acid (*e*.*g*. the sodium salt) or a mixture of two or more of such acids or salts. The complexing agent may comprise an inorganic acid such as nitric acid, halogenic acids, sulphuric acid, phosphoric acid or salts of such acids or a mixture of such acids. The complexing agent may also comprise a mixture of such organic and inorganic acids or their corresponding salts. The complexing agent for aluminium preferably forms a water-soluble complex with aluminium, and in particular removes alumina which is formed during the steam treatment step from the crystalline silicate. A particularly preferred complexing agent may comprise an amine, preferably ethylene diamine tetraacetic acid (EDTA) or a salt thereof, in particular the sodium salt thereof. In a preferred embodiment, the framework silicon/aluminium ratio is increased by this process to a value of from about 150 to 1000, more preferably at least 200.

Following the aluminium leaching step, the crystalline silicate may be subsequently washed, for example with distilled water, and then dried, preferably at an elevated temperature, for example around 110°C.

Additionally, if during the preparation of the catalysts of the invention alkaline or alkaline earth metals have been used, the molecular sieve might be subjected to an ion-exchange step. Conventionally, ion-exchange is done in aqueous solutions using ammonium salts or inorganic acids.

Following the de-alumination step, the catalyst is thereafter calcined, for example at a temperature of from 400 to 800°C at atmospheric pressure for a period of from 1 to 10 hours.

In another specific embodiment the crystalline silicate catalyst is mixed with a binder, preferably an inorganic binder, and shaped to a desired shape, *e*.*g*. pellets. The binder is selected so as to be resistant to the temperature and other conditions employed in the dehydration process of the invention. The binder is an inorganic material selected from clays, silica, metal silicates, metal oxides such as ZrO₂ and/or metals, or gels including mixtures of silica and metal oxides. The binder is preferably alumina-free. If the binder which is used in conjunction with the crystalline silicate is itself catalytically active, this may alter the conversion and/or the selectivity of the catalyst. Inactive materials for the binder may suitably serve as diluents to control the amount of conversion so that products can be obtained economically and orderly without employing other means for controlling the reaction rate. It is desirable to provide a catalyst having a good crush strength. This is because in commercial use, it is desirable to prevent the catalyst from breaking down into powder-like materials. Such clay or oxide binders have been employed normally only for the purpose of improving the crush strength of the catalyst. A particularly preferred binder for the catalyst of the present invention comprises silica. The relative proportions of the finely divided crystalline silicate material and the inorganic oxide matrix of the binder can vary widely. Typically, the binder content ranges from 5 to 95% by weight, more typically from 20 to 50% by weight, based on the weight of the composite catalyst. Such a mixture of crystalline silicate and an inorganic oxide binder is referred to as a formulated crystalline silicate. In mixing the catalyst with a binder, the catalyst may be formulated into pellets, extruded into other shapes, or formed into spheres or a spray-dried powder. Typically, the binder and the crystalline silicate catalyst are mixed together by a mixing process. In such a process, the binder, for example silica, in the form of a gel is mixed with the crystalline silicate catalyst material and the resultant mixture is extruded into the desired shape, for example cylindic or multi-lobe bars. Spherical shapes can be made in rotating granulators or by oil-drop technique. Small spheres can further be made by spray-drying a catalyst-binder suspension. Thereafter, the formulated crystalline silicate is calcined in air or an inert gas, typically at a temperature of from 200 to 900°C for a period of from 1 to 48 hours. The binder preferably does not contain any aluminium compounds, such as alumina. This is because as mentioned above the preferred catalyst for use in the invention is de-aluminated to increase the silicon/aluminium ratio of the crystalline silicate. The presence of alumina in the binder yields other excess alumina if the binding step is performed prior to the aluminium extraction step. If the aluminium-containing binder is mixed with the crystalline silicate catalyst following aluminium extraction, this re-aluminates the catalyst.

In addition, the mixing of the catalyst with the binder may be carried out either before or after the steaming and extraction steps.

**According to a second advantageous embodiment the catalyst (A1) is** a crystalline silicate catalyst having a monoclinic structure, which has been produced by a process comprising providing a crystalline silicate of the MFI-type having a silicon/aluminium atomic ratio lower than 80; treating the crystalline silicate with steam and thereafter leaching aluminium from the zeolite by contact with an aqueous solution of a leachant to provide a silicon/aluminium atomic ratio in the catalyst of at least 180 whereby the catalyst has a monoclinic structure.

Preferably, in the steam treatment step the temperature is from 425 to 870°C, more preferably from 540 to 815°C, and at a water partial pressure of from 13 to 200kPa.

Preferably, the aluminium is removed by leaching to form an aqueous soluble compound by contacting the zeolite with an aqueous solution of a complexing agent for aluminium which tends to form a soluble complex with alumina.

In accordance with this preferred process for producing monoclinic crystalline silicate, the starting crystalline silicate catalyst of the MFI-type has an orthorhombic symmetry and a relatively low silicon/aluminium atomic ratio which can have been synthesized without any organic template molecule and the final crystalline silicate catalyst has a relatively high silicon/aluminium atomic ratio and monoclinic symmetry as a result of the successive steam treatment and aluminium removal. After the aluminium removal step, the crystalline silicate may be ion exchanged with ammonium ions. It is known in the art that such MFI-type crystalline silicates exhibiting orthorhombic symmetry are in the space group Pnma. The x-ray diffraction diagram of such an orthorhombic structure has one peak at d = around 0.365nm, d = around 0.305nm and d= around 0.300 nm (see EP-A-0146524).

The starting crystalline silicate has a silicon/aluminium atomic ratio lower than 80. A typical ZSM-5 catalyst has 3.08 wt% Al₂O₃, 0.062 wt% Na₂O, and is 100% orthorhombic. Such a catalyst has a silicon/aluminium atomic ratio of 26.9.

The steam treatment step is carried out as explained above. The steam treatment tends to reduce the amount of tetrahedral aluminium in the crystalline silicate framework by forming alumina. The aluminium leaching or extraction step is carried out as explained above. In the aluminium leaching step, the crystalline silicate is immersed in the acidic solution or a solution containing the complexing agent and is then preferably heated, for example heated at reflux conditions (at boiling temperature with total return of condensed vapours), for an extended period of time, for example 18 hours. Following the aluminium leaching step, the crystalline silicate is subsequently washed, for example with distilled water, and then dried, preferably at an elevated temperature, for example around 110°C. Optionally, the crystalline silicate is subjected to ion exchange with ammonium ions, for example by immersing the crystalline silicate in an aqueous solution of NH₄Cl.

Finally, the catalyst is calcined at an elevated temperature, for example at a temperature of at least 400°C. The calcination period is typically around 3 hours.

The resultant crystalline silicate has monoclinic symmetry, being in the space group P2₁/n. The x-ray diffraction diagram of the monoclinic structure exhibits three doublets at d = around 0.36, 0.31 and 0.19nm. The presence of such doublets is unique for monoclinic symmetry. More particularly, the doublet at d = around 0.36, comprises two peaks, one at d = 0.362nm and one at d = 0.365nm. In contrast, the orthorhombic structure has a single peak at d = 0.365nm.

The presence of a monoclinic structure can be quantified by comparing the x-ray diffraction line intensity at d = around 0.36nm. When mixtures of MFI crystalline silicates with pure orthorhombic and pure monoclinic structure are prepared, the composition of the mixtures can be expressed as a monoclinicity index (in%). The x-ray diffraction patterns are recorded and the peak height at d=0.362nm for monoclinicity and d=0.365nm for orthorhombicity is measured and are denoted as Im and lo respectively. A linear regression line between the monoclinicity index and the Im/Io gives the relation needed to measure the monoclinicity of unknown samples. Thus the monoclinicity index % = (axlm/lob)x100, where a and b are regression parameters.

The such monoclinic crystalline silicate can be produced having a relatively high silicon/aluminium atomic ratio of at least 100, preferably greater than about 200 preferentially without using an organic template molecule during the crystallisation step. Furthermore, the crystallite size of the monoclinic crystalline silicate can be kept relatively low, typically less than 1 micron, more typically around 0.5 microns, since the starting crystalline silicate has low crystallite size which is not increased by the subsequent process steps. Accordingly, since the crystallite size can be kept relatively small, this can yield a corresponding increase in the activity of the catalyst. This is an advantage over known monoclinic crystalline silicate catalysts where typically the crystallite size is greater than 1 micron as they are produced in presence of an organic template molecule and directly having a high Si/Al ratio which inherently results in larger crystallites sizes.

**According to a third advantageous embodiment the catalyst (A1) is** a P-modified zeolite (Phosphorus-modified zeolite). Said phosphorus modified molecular sieves can be prepared based on MFI, MOR, MEL, clinoptilolite or FER crystalline aluminosilicate molecular sieves having an initial Si/Al ratio advantageously between 4 and 500. The P-modified zeolites of this recipe can be obtained based on cheap crystalline silicates with low Si/Al ratio (below 30).

By way of example said P-modified zeolite is made by a process comprising in that order:
- selecting a zeolite (advantageously with Si/Al ratio between 4 and 500) among H⁺ or NH₄⁺-form of MFI, MEL, FER, MOR, clinoptilolite;
- introducing P at conditions effective to introduce advantageously at least 0.05 wt% of P;
- separation of the solid from the liquid if any;
- an optional washing step or an optional drying step or an optional drying step followed by a washing step;
- a calcination step; the catalyst of the XTO and the catalyst of the OCP being the same or different.
   The zeolite with low Si/Al ratio has been made previously with or without direct addition of an organic template.

Optionally the process to make said P-modified zeolite comprises the steps of steaming and leaching. The method consists in steaming followed by leaching. It is generally known by the persons in the art that steam treatment of zeolites, results in aluminium that leaves the zeolite framework and resides as aluminiumoxides in and outside the pores of the zeolite. This transformation is known as dealumination of zeolites and this term will be used throughout the text. The treatment of the steamed zeolite with an acid solution results in dissolution of the extra-framework aluminiumoxides. This transformation is known as leaching and this term will be used throughout the text. Then the zeolite is separated, advantageously by filtration, and optionally washed. A drying step can be envisaged between filtering and washing steps. The solution after the washing can be either separated, by way of example, by filtering from the solid or evaporated.
P can be introduced by any means or, by way of example, according to the recipe described in US 3,911,041, US 5,573,990 and US 6,797,851.
The catalyst (A1) made of a P-modified zeolite can be the P-modified zeolite itself or it can be the P-modified zeolite formulated into a catalyst by combining with other materials that provide additional hardness or catalytic activity to the finished catalyst product.

The separation of the liquid from the solid is advantageously made by filtering at a temperature between 0-90°C, centrifugation at a temperature between 0-90°C, evaporation or equivalent.

Optionally, the zeolite can be dried after separation before washing. Advantageously said drying is made at a temperature between 40-600°C, advantageously for 1-10h. This drying can be processed either in a static condition or in a gas flow. Air, nitrogen or any inert gases can be used.

The washing step can be performed either during the filtering (separation step) with a portion of cold (<40°C) or hot water (> 40 but <90°C) or the solid can be subjected to a water solution (1 kg of solid/4 liters water solution) and treated under reflux conditions for 0.5-10 h followed by evaporation or filtering.

Final calcination step is performed advantageously at the temperature 400-700°C either in a static condition or in a gas flow. Air, nitrogen or any inert gases can be used.

**According to a specific embodiment of this third advantageous embodiment of the invention** the phosphorous modified zeolite is made by a process comprising in that order :
- selecting a zeolite ( advantageously with Si/Al ratio between 4 and 500, from 4 to 30 in a specific embodiment) among H⁺ or NH₄⁺-form of MFI, MEL, FER, MOR, clinoptilolite;
- steaming at a temperature ranging from 400 to 870°C for 0.01-200h;
- leaching with an aqueous acid solution at conditions effective to remove a substantial part of Al from the zeolite;
- introducing P with an aqueous solution containing the source of P at conditions effective to introduce advantageously at least 0.05 wt% of P;
- separation of the solid from the liquid;
- an optional washing step or an optional drying step or an optional drying step followed by a washing step;
- a calcination step.

Optionally between the steaming step and the leaching step there is an intermediate step such as, by way of example, contact with silica powder and drying.

Advantageously the selected MFI, MEL, FER, MOR, clinoptilolite (or H⁺ or NH₄⁺-form MFI, MEL, FER, MOR, clinoptilolite) has an initial atomic ratio Si/Al of 100 or lower and from 4 to 30 in a specific embodiment. The conversion to the H⁺ or NH₄⁺-form is known per se and is described in US 3911041 and US 5573990.

Advantageously the final P-content is at least 0.05 wt% and preferably between 0.3 and 7 w%. Advantageously at least 10% of Al, in respect to parent zeolite MFI, MEL, FER, MOR and clinoptilolite, have been extracted and removed from the zeolite by the leaching.

Then the zeolite either is separated from the washing solution or is dried without separation from the washing solution. Said separation is advantageously made by filtration. Then the zeolite is calcined, by way of example, at 400°C for 2-10 hours.

In the steam treatment step, the temperature is preferably from 420 to 870°C, more preferably from 480 to 760°C. The pressure is preferably atmospheric pressure and the water partial pressure may range from 13 to 100 kPa. The steam atmosphere preferably contains from 5 to 100 vol % steam with from 0 to 95 vol % of an inert gas, preferably nitrogen. The steam treatment is preferably carried out for a period of from 0.01 to 200 hours, advantageously from 0.05 to 200 hours, more preferably from 0.05 to 50 hours. The steam treatment tends to reduce the amount of tetrahedral aluminium in the crystalline silicate framework by forming alumina.

The leaching can be made with an organic acid such as citric acid, formic acid, oxalic acid, tartaric acid, malonic acid, succinic acid, glutaric acid, adipic acid, maleic acid, phthalic acid, isophthalic acid, fumaric acid, nitrilotriacetic acid, hydroxyethylenediaminetriacetic acid, ethylenediaminetetracetic acid, trichloroacetic acid trifluoroacetic acid or a salt of such an acid (*e*.*g*. the sodium salt) or a mixture of two or more of such acids or salts. The other inorganic acids may comprise an inorganic acid such as nitric acid, hydrochloric acid, methansulfuric acid, phosphoric acid, phosphonic acid, sulfuric acid or a salt of such an acid (*e*.*g*. the sodium or ammonium salts) or a mixture of two or more of such acids or salts.

The residual P-content is adjusted by P-concentration in the aqueous acid solution containing the source of P, drying conditions and a washing procedure if any. A drying step can be envisaged between filtering and washing steps.

Said P-modified zeolite can be used as itself as a catalyst. In another embodiment it can be formulated into a catalyst by combining with other materials that provide additional hardness or catalytic activity to the finished catalyst product. Materials which can be blended with the P-modified zeolite can be various inert or catalytically active materials, or various binder materials. These materials include compositions such as kaolin and other clays, various forms of rare earth metals, phosphates, metal silicates, alumina or alumina sol, titania, zirconia, quartz, silica or silica sol, and mixtures thereof. These components are effective in densifying the catalyst and increasing the strength of the formulated catalyst. The catalyst may be formulated into pellets, spheres, extruded into other shapes, or formed into a spray-dried particles. The amount of P-modified zeolite which is contained in the final catalyst product ranges from 10 to 90 weight percent of the total catalyst, preferably 20 to 70 weight percent of the total catalyst.

**As regards step c)** when the conversion of ethanol to hydrocarbons is 92 % and above (on a carbon basis) ethylene is around 50 to 55%, propylene around 5%, C4+ olefins around 20 to 30%, various hydrocarbons around 11 to 13%. Said various hydrocarbons are essentially aromatics around 1 to 3% and fuel around 10%.

**As regards step d), the fractionation of said effluent of step c)** removes water, unconverted ethanol, optionally the inert component, optionally the propylene and optionally the whole or a part of the various hydrocarbons to get a stream (D) comprising essentially ethylene, olefins having 4 carbon atoms or more (C4+ olefins) and optionally the inert component. The fractionation is carried out by any means, they are known per se. The stream (D) comprises the ethylene, the C4+ olefins, the various hydrocarbons (in whole or in part), optionally the propylene and optionally the inert component.

"to remove optionally the inert component" has to be understood as follows :
If there is no inert component introduced at step a) it is clear that said inert component is not present in the effluent of step c) and not present in stream (D),
If an inert component is introduced at step a) there is in fractionation of step d) an option to remove it, thereby said inert component is not present in stream (D) or to let it, thereby said inert component is present in stream (D).

To remove only water and the unconverted ethanol is easy because they are soluble in water and the remaining components of the effluent of step c) are not soluble in water. The fractionation is thereby easy. To remove water, unconverted ethanol and the inert component requires more equipment but the stream (D) is reduced and thereby the OCP reactor.

Advantageously (D) comprises only the C4+ olefins and the ethylene.
More advantageously (D) comprises only the C4+ olefins and about 50 w% or less of ethylene. It means that if the concentration of ethylene in the effluent of step c) is too high a part of the ethylene has to be separated and sent to an appropriate recovery unit or recycled e.g. at step a).

**As regards stream (D1) of step e),** it may comprise any kind of olefin-containing hydrocarbon stream. (D1) may typically comprise from 10 to 100wt% olefins and furthermore may be fed undiluted or diluted by a diluent, the diluent optionally including a non-olefinic hydrocarbon. In particular, (D1) may be a hydrocarbon mixture containing normal and branched olefins in the carbon range C₄ to C₁₀, more preferably in the carbon range C₄ to C₆, optionally in a mixture with normal and branched paraffins and/or aromatics in the carbon range C₄ to C₁₀. Typically, the olefin-containing stream has a boiling point of from around -15 to around 180°C.

In particularly preferred embodiments of the present invention, (D1) comprises C₄ mixtures from refineries and steam cracking units. Such steam cracking units crack a wide variety of feedstocks, including ethane, propane, butane, naphtha, gas oil, fuel oil, *etc*. Most particularly, (D1) may comprise a C₄ cut from a fluidized-bed catalytic cracking (FCC) unit in a crude oil refinery which is employed for converting heavy oil into gasoline and lighter products. Typically, such a C₄ cut from an FCC unit comprises around 30-70wt% olefin. Alternatively, (D1) may comprise a C₄ cut from a unit within a crude oil refinery for producing methyl tert-butyl ether (MTBE) or ethyl tert-butyl ether (ETBE) which is prepared from methanol or ethanol and isobutene. Again, such a C₄ cut from the MTBE/ETBE unit typically comprises around 50wt% olefin. These C₄ cuts are fractionated at the outlet of the respective FCC or MTBE/ETBE unit. (D1) may yet further comprise a C₄ cut from a naphtha steam-cracking unit of a petrochemical plant in which naphtha, comprising C₅ to C₉ species having a boiling point range of from about 15 to 180°C, is steam cracked to produce, *inter alia*, a C₄ cut. Such a C₄ cut typically comprises, by weight, 40 to 50% 1,3-butadiene, around 25% isobutylene, around 15% butene (in the form of but-1-ene and/or but-2-ene) and around 10% n-butane and/or isobutane. (D1) may also comprise a C₄ cut from a steam cracking unit after butadiene extraction (raffinate 1), or after butadiene hydrogenation.

(D1) may yet further alternatively comprise a hydrogenated butadiene-rich C₄ cut, typically containing greater than 50wt% C₄ as an olefin. Alternatively, (D1) could comprise a pure olefin feedstock which has been produced in a petrochemical plant.

(D1) may yet further alternatively comprise light cracked naphtha (LCN) (otherwise known as light catalytic cracked spirit (LCCS)) or a C₅ cut from a steam cracker or light cracked naphtha, the light cracked naphtha being fractionated from the effluent of the FCC unit, discussed hereinabove, in a crude oil refinery. Both such feedstocks contain olefins. (D1) may yet further alternatively comprise a medium cracked naphtha from such an FCC unit or visbroken naphtha obtained from a visbreaking unit for treating the residue of a vacuum distillation unit in a crude oil refinery.

Advantageously the mixture of (D) and (D1) sent to the OCP in step f) contains at least 20% of C4+ olefins and less than about 50 wt% of ethylene.

**As regards the reaction in step f),** it is referred as an "OCP process". It can be any catalyst provided it is selective to light olefins. Said OCP process is known per se. It has been described in EP 1036133, EP 1035915, EP 1036134, EP 1036135, EP 1036136, EP 1036138, EP 1036137, EP 1036139, EP 1194502, EP 1190015, EP 1194500 and EP 1363983 the content of which are incorporated in the present invention.

The catalyst can be selected among the catalysts (A1) of step b) above and is employed under particular reaction conditions whereby the catalytic cracking of the C₄⁺ olefins readily proceeds. Different reaction pathways can occur on the catalyst. Olefinic catalytic cracking may be understood to comprise a process yielding shorter molecules via bond breakage.

In the catalytic cracking process of the OCP reactor, the process conditions are selected in order to provide high selectivity towards propylene or ethylene, as desired, a stable olefin conversion over time, and a stable olefinic product distribution in the effluent. Such objectives are favoured with a low pressure, a high inlet temperature and a short contact time, all of which process parameters are interrelated and provide an overall cumulative effect.

The process conditions are selected to disfavour hydrogen transfer reactions leading to the formation of paraffins, aromatics and coke precursors. The process operating conditions thus employ a high space velocity, a low pressure and a high reaction temperature. The LHSV ranges from 0.5 to 30 hr⁻¹, preferably from 1 to 30 hr⁻¹. The olefin partial pressure ranges from 0.1 to 2 bars, preferably from 0.5 to 1.5 bars (absolute pressures referred to herein). A particularly preferred olefin partial pressure is atmospheric pressure (i.e. 1 bar). The mixture of (D) and (D1) is preferably fed at a total inlet pressure sufficient to convey the feedstocks through the reactor. Said feedstock (the mixture of (D) and (D1)) may be fed undiluted or diluted in an inert gas, e.g. nitrogen or steam. Preferably, the total absolute pressure in the reactor ranges from 0.5 to 10 bars. The use of a low olefin partial pressure, for example atmospheric pressure, tends to lower the incidence of hydrogen transfer reactions in the cracking process, which in turn reduces the potential for coke formation which tends to reduce catalyst stability. The cracking of the olefins is preferably performed at an inlet temperature of the feedstock of from 400° to 650°C, more preferably from 450° to 600°C, yet more preferably from 540°C to 590°C. In order to maximize the amount of ethylene and propylene and to minimize the production of methane, aromatics and coke, it is desired to minimize the presence of diolefins in the feed. Diolefin conversion to monoolefin hydrocarbons may be accomplished with a conventional selective hydrogenation process such as disclosed in U.S. Pat. No. 4,695,560 hereby incorporated by reference.

The OCP reactor can be a fixed bed reactor, a moving bed reactor or a fluidized bed reactor. A typical fluid bed reactor is one of the FCC type used for fluidized-bed catalytic cracking in the oil refinery. A typical moving bed reactor is of the continuous catalytic reforming type. As described above, the process may be performed continuously using a pair of parallel "swing" reactors. The mixture of

(D) and (D1) cracking process is endothermic; therefore, the reactor should be adapted to supply heat as necessary to maintain a suitable reaction temperature. Several reactors may be used in series with interheating between the reactors in order to supply the required heat to the reaction. Each reactor does a part of the conversion of the feedstock. Online or periodic regeneration of the catalyst may be provided by any suitable means known in the art.

The various preferred catalysts of the OCP reactor have been found to exhibit high stability, in particular being capable of giving a stable propylene yield over several days, *e*.*g*. up to ten days. This enables the olefin cracking process to be performed continuously in two parallel "swing" reactors wherein when one reactor is in operation, the other reactor is undergoing catalyst regeneration. The catalyst can be regenerated several times.

**As regards step g) and the effluent of OCP reactor of step f),** said effluent comprises methane, ethylene, propylene, optionally the inert component and hydrocarbons having 4 carbon atoms or more. Advantageously said OCP reactor effluent is sent to a fractionator and the light olefins (ethylene and propylene) are recovered. Advantageously the hydrocarbons having 4 carbon atoms or more are recycled at the inlet of the OCP reactor. Advantageously, before recycling said hydrocarbons having 4 carbon atoms or more at the inlet of the OCP reactor, said hydrocarbons having 4 carbon atoms or more are sent to a second fractionator to purge the heavies.
Optionally, in order to adjust the propylene to ethylene ratio, ethylene in whole or in part can be recycled over the OCP reactor and advantageously converted into more propylene. Ethylene can also be recycled in whole or in part at the inlet of the reactor (A).

**As regards the second embodiment of the present invention** the detailed description is the same as explained above except the catalyst in reactor (A) which is :
- a crystalline silicate having a ratio Si/Al of at least about 100, or
- a dealuminated crystalline silicate, or
- a phosphorus modified zeolite.

These catalysts have been described above.

### [Examples]

### Example I

This catalyst comprises a commercially available silicalite (S115 from UOP, Si/Al=150) which had been subjected to a dealumination treatment by combination of steaming with acid treatment so as provide Si/Al ratio 270. Then the dealuminated zeolite was extruded with silica as binder to have 70% of zeolite in the granule. A detailed procedure of catalyst preparation is described in EP 1194502 B1 (Example I).

### Example II

### Ethanol conversion in reactor (A)

Catalyst tests were performed on 10 ml (6,3 g) of catalyst grains (35-45 meshes) loaded in a tubular reactor with internal diameter 11 mm. A mixture 95 wt% ethanol + 5wt% water was subjected to a contact with catalyst described in the example I in a fixed bed reactor at 380°C, LHSV = 7h⁻¹ P=4 barg. The results are given in table 1 hereunder. The values are the weight percents on carbon basis.

### Example III (comparative)

### Ethanol conversion in reactor (A)

Catalyst tests were performed on 10 ml (6,3 g) of catalyst grains (35-45 meshes) loaded in the tubular reactor with internal diameter 11 mm. A mixture 95 wt% ethanol + 5wt% water was subjected to a contact with catalyst described in the example I in a fixed bed reactor at 380°C, LHSV = 7h⁻¹ P=0.35 barg. The results are given in table 1 hereunder. The values are the weight percents on carbon basis.

**Table I**

| | Ex II | Ex III |
|---|---|---|
| | Ethanol to HC* Dehydration/oligomerization | Ethanol dehydration |
| EtOH conv to HC*, % | 94.4 | 99.5 |
| Yield on C-basis, wt% | | |
| C1 (methane) | 0.01 | 0.00 |
| C2-ethylene | 52.6 | 97.0 |
| C3-propylene | 5.3 | 0.4 |
| C4+ olefins | 25.6 | 1.8 |
| Aromatics | 1.3 | 0.0 |
| Paraffin's | 9.6 | 0.3 |

| | | |
|---|---|---|
| *HC- hydrocarbons | | |

The above data demonstrate a possibility to convert substantially all ethanol to hydrocarbon feedstock at low temperature.

### Example IV

### OCP reaction (OCP reactor)

A feedstock containing 40wt% of C2- (ethylene), 36.6 wt% of C4-(olefins) and 26.4 wt% of C4 paraffin's was subjected for cracking in tubular reactor with internal diameter 11 mm (same as in previous ex II) over the catalyst described in the example I (560°C, WHSV= 11h⁻¹, P=0.5 barg). This feedstock represents the case if around 55 wt% of ethylene produced in the first reactor (A) after propylene and water extraction were blended with C4+ hydrocarbons from the same effluent described in the example II optionally diluted with paraffin's were sent to cracking reactor and the weight ratio C4-/C2- was maintained during recycle at 0.8. The total results comprising the sum of the OCP single-pass effluent, propylene produced in the first reactor and non-reacted ethylene are in table 2 hereunder. The values in the table are given in the weight percent on carbon basis and represent an average catalyst performance during 10h TOS.

### Example V (Comparative )

Ethylene to heavy olefins (feeding of ethylene to the OCP reactor under OCP conditions)
Catalyst tests were performed on 10 ml (6,3 g) of catalyst grains (35-45 meshes) loaded in the tubular reactor with internal diameter 11 mm. A pure ethylene was subjected to a contact with catalyst described in the example I in a fixed bed reactor at 560°C, LHSV = 11h⁻¹ P=0.5 barg. The results are given in table 3 hereunder. The values in the table are given in the weight percent on carbon basis dry basis and represent an average catalyst performance during 10h TOS.

### Example VI (Comparative)

Ethanol to olefins (direct conversion of ethanol in the OCP reactor at OCP conditions)
Catalyst tests were performed on 10 ml (6,3 g) of catalyst grains (35-45 meshes) loaded in the tubular reactor with internal diameter 11 mm. A pure ethanol was subjected to a contact with catalyst described in the example I in a fixed bed reactor at 550°C, LHSV = 10h⁻¹ P=0.5 barg. The results are given in table 2 hereunder. The values in the table are given in the weight percent on carbon basis dry basis and represent an average catalyst performance during 10h TOS.

**Table 2**

| | Ex IV | Ex V : Comparative | Ex VI : Comparative |
|---|---|---|---|
| Process | EtOH to HC* + OCP (single pass) : A part of (D) is mixed with (D1) and is reacted in the OCP reactor | EtOH to ethylene+ OCP (single pass) | EtOH to olefins (single pass). EtOH is reacted in the OCP reactor |
| Conversion of EtOH to HC* | 94,4 (first + second reactor) | 99,5 (first + second reactor) | 93,7 |
| Feed of the OCP | Ethylene + C4⁺olefins from the first reactor effluent (Ex II) | Ethylene from the first reactor effluent (Ex III) | EtOH |
| Yield on C-basis, wt% | | | |
| C1 (methane) | 0.2 | 0.1 | 0.4 |
| C2-ethylene | 44.4 | 88.3 | 82.4 |
| C3-propylene | 19.7 | 4.2 | 4.5 |
| Aromatics | 2.5 | 0.2 | 1.4 |
| C4+olefins | 17.0 | 6.2 | 3.0 |
| Paraffin's | 10.1 | 0.7 | 2.0 |

The data given above illustrate that the conversion of ethanol to hydrocarbon in the first reactor to a mixture of ethylene and C4+ olefins followed by water removal and sending of hydrocarbons feedstock to the OCP reactor is beneficial in term of propylene production in respect to direct conversion of (bio-) ethanol or (bio-) ethylene in OCP reactor under OCP conditions. Moreover this offers a solution to combine the endothermic process of ethanol dehydration and ethylene oligomerization in the first reactor and in the second reactor endothermic cracking with exothermic ethylene transformation to heavies.

## Claims

1. Process for the conversion of ethanol to make essentially ethylene and propylene, comprising :
a) introducing in a reactor (A) (also called the first low temperature reaction zone) a stream comprising ethanol under a partial pressure at least about 0.2 MPa, optionally water, optionally an inert component,
b) contacting said stream with a catalyst (A1) in said reactor (A) at conditions effective to convert at least a portion of the ethanol to essentially ethylene, propylene and olefins having 4 carbon atoms or more (C4+ olefins),
c) recovering from said reactor an effluent comprising : ethylene and C4+ fraction containng mainly olefins having 4 carbon atoms or more (C4+ olefins), propylene and various hydrocarbons, water, optionally unconverted ethanol and the optional inert component of step a),
d) fractionating said effluent of step c) to remove water, unconverted ethanol, optionally the inert component, optionally the propylene and optionally the whole or a part of the various hydrocarbons to get a stream (D) comprising essentially ethylene, olefins having 4 carbon atoms or more (C4+ olefins) and optionally the inert component,
e) introducing at least a part of said stream (D) optionally mixed with a stream (D1) comprising olefins having 4 carbon atoms or more (C4+ olefins) in a OCP reactor (also called the second high temperature reaction zone) under the condition that said mixture (D)+(D1) comprises at least 10wt% of C4+ olefins,
f) contacting said stream comprising at least a part of (D) and the optional (D1) in said OCP reactor with a catalyst which is selective towards light olefins in the effluent, to produce an effluent with an olefin content of lower molecular weight than that of the feedstock,
g) fractionating said effluent of step f) to produce at least an ethylene stream, a propylene stream and a fraction consisting essentially of hydrocarbons having 4 carbon atoms or more, optionally recycling ethylene in whole or in part at the inlet of the OCP reactor of step f), or at the inlet of the reactor (A) or in part at the inlet of the OCP reactor of step f) and in part at the inlet of the reactor (A), optionally recycling the fraction consisting essentially of hydrocarbons having 4 carbon atoms or more at the inlet of the OCP reactor.

2. Process according to claim 1 wherein the WHSV of ethanol in step b) is from 0.1 to 20 h⁻¹.

3. Process according to claim 2 wherein the WHSV of ethanol in step b) is from 0.4 to 20 h⁻¹.

4. Process according to any one of the preceding claims wherein the catalyst (A1) and catalyst in the OCP reactor of step f) are selected among the crystalline silicates and the phosphorus modified zeolites.

5. Process according to claim 4 wherein the crystalline silicates as catalyst (A1) are selected among the crystalline silicates having a ratio Si/Al of at least about 100 and the dealuminated crystalline silicates.

6. Process according to claim 5 wherein the crystalline silicate having a ratio Si/Al of at least about 100 and the dealuminated crystalline silicate are selected among the MFI, MEL, FER, MTT, MWW, TON, EUO, MFS and ZSM-48 family of microporous materials consisting of silicon, aluminium, boron and oxygen.

7. Process according to claim 6 wherein the crystalline silicate having a ratio Si/Al of at least about 100 is selected among the MFI and the MEL.

8. Process according to any one of claims 5 to 7 wherein the Si/Al ratio of the crystalline silicate ranges from 100 to 1000.

9. Process according to any one of claims 5 to 8 wherein the crystalline silicate having a ratio Si/Al of at least about 100 or the dealuminated crystalline silicate is steamed to remove aluminium from the crystalline silicate framework.

10. Process according to claim 9 wherein, further to the steaming, aluminium is extracted from the catalyst by contacting the catalyst with a complexing agent for aluminium to remove from pores of the framework alumina deposited therein during the steaming step thereby to increase the silicon/aluminium atomic ratio of the catalyst.

11. Process according to any one of the preceding claims wherein the temperature of the OCP reactor ranges from 540°C to 590°C.

12. Process for the conversion of ethanol to make essentially ethylene, olefins having 4 carbon atoms or more (C4+ olefins) and minor amounts of propylene, comprising :
a) introducing in a reactor (A) a stream comprising ethanol under a partial, pressure of at least 0.2 MPa, optionally water, optionally an inert component,
b) contacting said stream with a catalyst in said reactor (A) at conditions effective to convert at least a portion of the ethanol to essentially ethylene and C4+ fraction containng mainly olefins having 4 carbon atoms or more (C4+ olefins),
c) recovering from said reactor an effluent comprising : ethylene and olefins having 4 carbon atoms or more (C4+ olefins), propylene and various hydrocarbons, water, optionally unconverted ethanol and the optional inert component of step a),
Wherein the catalyst is :
• a crystalline silicate having a ratio Si/Al of at least about 100, or
• a dealuminated crystalline silicate, or
• a phosphorus modified zeolite, the temperature ranges from 280°C to 500°C.

13. Process according to claim 12 wherein the WHSV of ethanol in step b), ranges from 2 to 20 h⁻¹.

14. Process according to claim 13 wherein the WHSV of ethanol in step b), ranges from 4 to 20 h⁻¹.

15. Process according to any one of the preceding claims wherein the partial pressure of ethanol in steps a) and b) is from 0.20 MPa to 3 MPa,

16. Process according to claim 15 wherein the partial pressure of ethanol in steps a) and b) is from 0.35 MPa to 1 MPa.

17. Process according to any one of the preceding claims wherein the temperature in step b) ranges from 300°C to 400°C.
